# EUROPÄISCHE PATENTSCHRIFT

(11) **EP 0 864 556 B1**
(45) Veröffentlichungstag und Bekanntmachung des Hinweises auf die Patenterteilung: **28.03.2001**
(21) Anmeldenummer: 98101963.1
(22) Anmeldetag: 05.02.1998
(51) Int. Cl.: C07C 45/82, C07D 307/28, C07C 49/293

(54) **Verfahren zur Herstellung von hochreinem Cyclopropylmethylketon**
Process for the production of high purity cyclopropylmethylketone
Procédé pour la préparation de cyclopropylméthylcétone à haute pureté

(30) Priorität: 15.03.1997 DE 19710879
(43) Veröffentlichungstag der Anmeldung: 16.09.1998
(73) Patentinhaber: Degussa-Hüls Aktiengesellschaft, 60287 Frankfurt am Main (DE)
(72) Erfinder: Kleemiss, Wolfgang, Dr., 45721 Haltern (DE); Köhler, Günther, Dr., 45770 Marl (DE); Neumann, Manfred, Dr., 45770 Marl (DE)

(56) Entgegenhaltungen:
- EP-A- 0 552 586

## Beschreibung

Die Erfindung betrifft ein Verfahren zur Herstellung von hochreinem Cyclopropylmethylketon (CPMK) aus Reaktionsausträgen bzw. Gemischen, die CPMK, 4,5-Dihydro-2-methylfuran (DHMF), Additionsprodukte des DHMF sowie gegebenenfalls weitere Verunreinigungen enthalten, die aus der Synthese von CPMK aus 2-Acetylbutyrolacton (ABL) resultieren können.

CPMK ist ein wertvolles Zwischenprodukt, das für die Synthese von Agro- und Pharmawirkstoffen in hoher Reinheit benötigt wird. Für den Einsatz von CPMK als Synthesebaustein für den Agro- und Pharmabereich ist eine Reinheit von > 99 Gew.-% erforderlich.

CPMK kann besonders vorteilhaft aus ABL hergestellt werden, indem man ABL alkalihalogenidkatalysiert unter CO₂-Abspaltung zum CPMK cyclisiert [Chemistry Letters 11, S. 1149-52 (1975)]. Die Herstellung kann kontinuierlich durchgeführt werden, indem das Alkalihalogenid bei hoher Temperatur in einem Lösemittel vorgelegt und das ABL kontinuierlich hinzudosiert wird. Da bei der Reaktion neben dem Hauptprodukt CPMK auch DHMF mit einem Anteil von gewöhnlich 5 bis 30 Gew.-% entsteht, wird als Wertprodukt der Umsetzung zeitgleich zur Reaktion immer ein Gemisch aus CPMK und DHMF kontinuierlich abdestilliert. Entsprechende Herstellverfahren sind z. B. aus EP-A 0 552 586 oder DE-A 195 03 241 bekannt. Das so gewonnene CPMK erfüllt aber wegen des Gehalts an DHMF die genannten Reinheitsanforderungen nicht.

DHMF ist hinreichend leichtersiedend als CPMK und kann daher grundsätzlich problemlos destillativ vom CPMK abgetrennt werden, um CPMK hoher Reinheit zu erhalten.

Allerdings reagiert DHMF in Gegenwart von Säure mit zahlreichen Nukleophilen, wie Wasser, Alkoholen, Aminen etc., zu hochsiedenden Additionsprodukten (vgl. Houben/Weyl: Methoden der organischen Chemie, Bd. VI/3, 1965, S. 698). Bei der Addition von Wasser an DHMF entsteht z. B. Acetopropanol, das seinerseits wiederum an DHMF addieren kann. Diese Additionsreaktionen sind reversibel, so daß bei hohen Temperaturen Acetopropanol oder sein Additionsprodukt an DHMF bzw. andere Additionsprodukte an DHMF unter Eliminierung bzw. Cyclisierung wieder DHMF freisetzen können.

In einem Rohgemisch aus CPMK und DHMF sind immer auch geringe Wassermengen oder auch andere nukleophil reagierende Verunreinigungen vorhanden, die auf die oben geschilderte Weise DHMF reversibel in Hochsieder überführen. Eine Reindestillation von CPMK zur Abtrennung von DHMF führt daher abhängig von der Sumpftemperatur zur Rückspaltung der höhersiedenden DHMF-Additionsprodukte und damit erneut zu einer Verunreinigung von CPMK mit DHMF im Nachlauf der Destillation. Bei der Reindestillation von CPMK besteht also stets das Problem der Verunreiniung von CPMK durch Rückspaltung der Additionsprodukte von DHMF in Abhängigkeit von der Sumpftemperatur. Wegen der erforderlichen Sumpftemperaturen bei herkömmlichen Destillationen von 110 bis 180 °C ergibt sich zur Einhaltung der Reinheitsanforderungen die Notwendigkeit, weiteres DHMF aus den Fraktionen des Vorlaufs und des Nachlaufs der Destillation abzutrennen. Dieser Umstand erfordert aber einen hohen technischen Aufwand und geht zu Lasten der Ausbeute an CPMK.

Die Absenkung der Sumpftemperatur durch Anlegen von Vakuum und damit eine Verhinderung der Rückspaltung von DHMF-Additionsprodukten und der damit einhergehenden Verunreinigung von CPMK scheidet aus, da der technische Aufivand für die Kondensation des relativ tief siedenden CPMK zu groß ist.

Es besteht daher die Aufgabe, ein einfaches Verfahren zur Herstellung von hochreinem CPMK in sehr guten Ausbeuten bereitzustellen, bei dem als Einsatzmaterial Reaktionsausträge bzw. Gemische dienen, die CPMK, DHMF, Additionsprodukte des DHMF sowie gegebenenfalls weitere Verunreinigungen enthalten, die aus der Synthese von CPMK aus 2-Acetylbutyrolacton (ABL) resultieren können.

Diese Aufgabe wird erfindungsgemäß gelöst gemäß Patentanspruch 1 durch ein Verfahren zur Herstellung von hochreinem Cyclopropylmethylketon aus Reaktionsausträgen oder anderen Gemischen, die CPMK, DHMF, Additionsprodukte des DHMF und gegebenenfalls weitere Verunreinigungen enthalten, die aus der Synthese von CPMK aus ABL resultieren können, das gekennzeichnet ist durch zumindest einen kontinuierlich arbeitenden Rektifikationsschritt mit seitlichem Zulauf des durch Rektifikation zu zerlegenden Gemisches in eine Rektifikationsvorrichtung oberhalb des Sumpfes.

Überraschenderweise läßt sich mit Hilfe des erfindungsgemäßen Verfahrens hochreines CPMK mit einer Reinheit von > 99 Gew.-% in hoher Ausbeute herstellen. Es können erfindungsgemäß Ausbeuten an hochreinem CPMK von über 90 % (bezogen auf den Anteil an CPMK im erfindungsgemäß aufzuarbeitenden Ausgangsgemisch) erzielt werden.

Dies wird ermöglicht durch den erfindungsgemäßen kontinuierlich arbeitenden Rektifikationsschritt mit seitlichem Zulauf des rektifikativ zu zerlegenden Gemisches seitlich oberhalb des Sumpfes in die Rektifikationsvorrichtung, durch den die im Sumpf vorgelegte Menge des dabei zu zerlegenden Gemisches im Vergleich zu herkömmlichen, rein destillativen Verfahren deutlich verringert wird. Der erfindungsgemäße Rektifikationsschritt mit der Vorlage letztlich stets nur einer kleinen Teilmenge des erfindungsgemäß aufzuarbeitenden Ausgangsgemisches in der Sumpfblase ermöglicht eine Absenkung der Sumpftemperatur während der gesamten, möglicherweise weitere destillative Verfahrensschritte umfassenden Aufarbeitung des Ausgangsgemisches auf kleiner oder gleich 160 °C, bevorzugt kleiner oder gleich 140 °C, besonders bevorzugt kleiner oder gleich 120 °C. Dadurch wird die Rückspaltung von Additionsprodukten des DHMF zurückgedrängt und die Gewinnung von hochreinem CPMK erleichtert.

Das erfindungsgemäße Verfahren ist sehr einfach in seiner Durchführung, da für den erfindungsgemäßen Rektifikationsschritt herkömmliche Rektifikationsvorrichtungen eingesetzt werden können. Wie bereits erwähnt, kann das erfindungsgemäße Verfahren zur Aufarbeitung des Ausgangsgemisches neben diesem Rektifikationsschritt weitere - z. B. destillative - Verfahrensschritte umfassen.

In einer bevorzugten Ausführung des erfindungsgemäßen Verfahrens wird beispielsweise zunächst ein Teil des verglichen mit CPMK leichtersiedenden DHMF aus dem zu zerlegenden Ausgangsgemisch abdestilliert. Diese Destillation kann kontinuierlich oder diskontinuierlich bei Sumpftemperaturen vorzugsweise unter 120 °C erfolgen. Anschließend wird das entstehende, vor allem noch mit hochsiedenden Additionsprodukten des DHMF sowie mit weiteren Verbindungen, die aus der Synthese von CPMK aus 2-Acetylbutyrolacton (ABL) resultieren können, verunreinigte Roh-CPMK in eine Destillationskolonne seitlich oberhalb des Sumpfes eindosiert. Am Kopf der Kolonne wird im stationären Betrieb CPMK mit einer Reinheit > 99 Gew.-% abgenommen, wobei die Sumpftemperatur 160 °C, bevorzugt 140 °C, besonders bevorzugt 120°C, nicht übersteigt. Bezogen auf den CPMK-Gehalt im zu zerlegenden Ausgangsgemisch können destillative Ausbeuten an hochreinem CPMK von größer 95 % erreicht werden.

In einer weiteren besonders bevorzugten Ausführung umfaßt das erfindungsgemäße Verfahren lediglich einen einzigen kontinuierlich arbeitenden Rektifikationsschritt zur Herstellung von hochreinem CPMK. Das zu zerlegende Ausgangsgemisch kann der Rektifikationsvorrichtung dabei seitlich etwa in Kolonnenmitte derart zugeführt werden, daß das Verhältnis der Trennstufenzahl (Anzahl theoretischer Böden) des Verstärkungsteils der Kolonne zur Trennstufenzahl des Abtriebsteils einen Wert von 1 bis 2 annimmt. Am Kopf der Kolonne wird kontinuierlich das leichtsiedende DHMF abgetrennt, während in einem Seitenabzug kurz über dem Destillationssumpf das CPMK in hoher Reinheit > 99 Gew.-% und hoher Ausbeute von gewöhnlich über 90 % gewonnen werden kann. Die seitliche Entnahme des CPMK erfolgt vorzugsweise unmittelbar oberhalb der siedenden Flüssigkeit des Sumpfes; sie kann aber auch noch im sich in Sumpfnähe befindenden unteren Drittel - bezogen auf die Trennstufenzahl-der Abtriebssäule erfolgen. Bei Bedarf kann während der Destillation ein Teil des Sumpfproduktes abgezogen werden.

In der Praxis haben sich zur Durchführung dieser letztgenannten besonders bevorzugten Verfahrensausführung Destillationskolonnen mit geordneten Destillationspackungen bewährt. Gute Ergebnisse wurden z. B. mit einer Kolonne mit einer Trennleistung von 25 theoretischen Böden, angeordnet in 3 Schüssen, oder einer Kolonne mit einer Trennleistung von 35 theoretischen Böden, angeordnet in 4 Schüssen, erzielt. Die seitliche Entnahme des hochreinen CPMK erfolgte hier entweder unter oder über der 1. Packung oberhalb des Sumpfes; der Zulauf war entweder mittig oder sumpfnah oberhalb der ersten der insgesamt 3 Packungen angeordnet. Die zuletzt beschriebene einstufige bevorzugte Ausführung ist wegen ihrer einfachen Handhabung und Durchführbarkeit insbesondere für die großtechnische Anwendung geeignet.

Das erfindungsgemäße Verfahren wird zweckmäßigerweise unter Normaldruck durchgeführt, kann aber auch bei einem Druck von 300 bis 1 050 mbar, vorzugsweise bei 500 bis 1 030 mbar, besonders bevorzugt bei 800 bis 1 020 mbar, durchgeführt werden.

Das erfindungsgemäße Verfahren wird durch die nachfolgenden Beispiele näher erläutert, ohne darauf beschränkt zu sein:

### Vergleichsbeispiel:

- Destillationseinsatz:: 1 239,3 g
- Zusammensetzung:: CPMK: 1 080,7 g (87,2 Gew.-%)
DHMF: 137,6 g (11,1 Gew.-%)
Wasser: 7,4 g ( 0,6 Gew.-%)
- Apparatur:: 0,5 m Multifil-Destillationskolonne
2 1-Destillationskolben
- Destillation:: Das zu rektifizierende Gemisch wird in der Destillationsblase vorgelegt. Mit Hilfe eines Ölbades wird das Gemisch zum Sieden erhitzt. Zunächst wird bei einem Rücklaufverhältnis von 20 : 1 DHMF abdestilliert. Dann wird bein einem Rücklaufverhältnis von 5 : 1 > 99 Gew.-%iges CPMK gewonnen. Als Nachlauf erhält man bei erhöhter Sumpftemperatur eine mit DHMF verunreinigte CPMK-Fraktion.

Es werden die in Tabelle 1 aufgeführten Fraktionen erhalten.

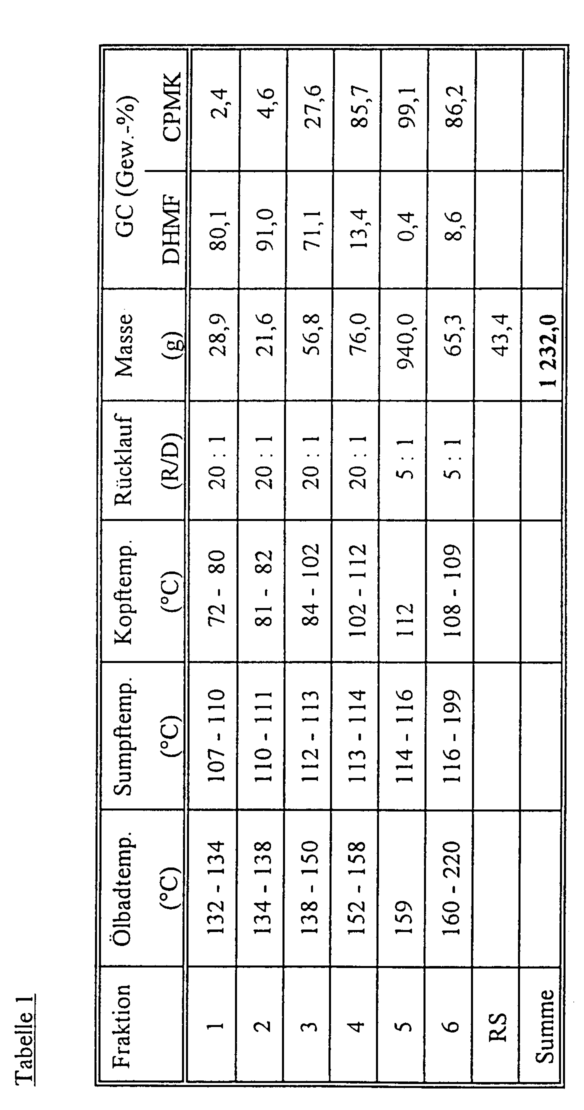

Fraktion 5 stellt das Wertprodukt dar. Die Destillationsausbeute an 99 Gew.-%igem CPMK beträgt 86 %, bezogen auf 1 080,7 Einsatzmenge an CPMK. Man erhält als Nachlauf eine CPMK-Fraktion, die wieder stark mit DHMF verunreinigt ist (Fraktion 6).

### Beispiel 1 (erfindungsgemäß, zweistufige Destillation):

- Destillationseinsatz:: 1 593,5 g
- Zusammensetzung:: CPMK: 1 362,4 g (85,5 Gew.-%)
DHMF: 200,8 g (12,6 Gew.-%)
Wasser: 15,9 g ( 1,0 Gew.-%)
- Apparatur:: 1 l-Destillationskolben mit seitlichem Ablauf, zwei aufeinandergesetzte, beheizbare 0,5 m Multifil-Kolonnen mit mittlerer Einspeisung

### a) DHMF/Wasser-Abtrennung

### Durchführung:

Ca. 500 g des Destillationseinsatzes werden in dem Destillationskolben vorgelegt. Bei einer Sumpftemperatur von 109 bis 113 °C und einem Rücklauf von 10 : 1 wird ein DHMF/Wasser-Gemisch zunächst bis zu einer Kopftemperatur von 83 °C abdestilliert. Anschließend wird der verbleibende Destillationseinsatz kontinuierlich mit einer Geschwindigkeit von ca. 150 g/h in die Mitte der Kolonne eindosiert. Der überlaufende Destillationssumpf wird in einem Vorratsgefäß gesammelt.

### Bedingungen der kontinuierlichen Destillation:

- Sumpftemperatur:: 113 - 114 °C
- Kopftemperatur:: 82 - 83 °C
- Rücklauf (R/D):: 12 : 1

Nach Abschluß der kontinuierlichen Destillation liegen vor:
1. Destillat: 214,5 g
   - Zusammensetzung:: CPMK: 20,3 g ( 9,5 Gew.-%)
   DHMF: 180,2 g (84,0 Gew.-%)
   Wasser: 14,0 g ( 6,5 Gew.-%)
2. Destillationssumpf: 1 378,0 g
   - Zusammensetzung:: CPMK: 1 342,0 g (97,4 Gew.-%)
   DHMF: 20,8 g ( 1,5 Gew.-%)
   Wasser: 1,9 g ( 0,1 Gew.-%)
   Rest: 13,3 g ( 1,0 Gew.-%)

### b) CPMK - Reindestillation (Rektifikation)

### Destillationseinsatz: Destillationssumpf der vorangegangenen Destillation (1 378,0 g)

### Durchführung:

In der oben beschriebenen Apparatur werden ca. 400 g des Destillationssumpfes der vorangegangenen DHMF/Wasser-Abtrennung vorgelegt, wobei der Sumpfablauf verschlossen wird. Man beginnt bei einer Sumpftemperatur von 113 °C und einer Kopftemperatur von 111 °C die Reindestillation und beginnt daraufhin mit der Eindosierung (150 g/h) des restlichen Destillationssumpfes in die Kolonnenmitte.

### Bedingungen der kontinuierlichen Rektifikation:

- Sumpftemperatur:: 113 - 114 °C
- Kopftemperatur:: 112 °C
- Rücklauf R/D:: 5 : 10

Man erhält als Ergebnis zwei Fraktionen:
- Fraktion 1:: 50,0 g enthaltend 98,0 Gew.-% DHMF und
1,5 Gew.-% CPMK
- Fraktion 2:: 1 287,0 g enthaltend 99,5 Gew.- % CPMK
- Rückstand:: 40,0 g

Damit beträgt die Ausbeute an 99,5 Gew.-%igem CPMK 94,4 % bezogen auf die Masse CPMK im ursprünglichen Destillationseinsatz.

### Beispiel 2 (erfindungsgemäß, einstufige Rektifikation):

- Destillationseinsatz:: 3 971,9 g
- Zusammensetzung:: CPMK: 3 654,1 g (92,0 Gew.-%)
DHMF: 278,1 g ( 7,0 Gew.-%)
Wasser: 35,7 g ( 0,9 Gew.-%)
- Apparatur:: 1 l-Destillations-Dreihalskolben mit zwei aufeinandergesetzten,beheizbaren 0,5 m - Multifil-Kolonnen mit mittlerer Einspeisung und direkt über dem Sumpf aufgesetzter Destillationsbrücke.

### Durchführung:

Bei einer Ölbadtemperatur von 128 °C wird der Destillationseinsatz mit einer Geschwindigkeit von 150 g/h in die Mitte der Kolonne dosiert. Am Kopf der Kolonne wird DHMF abgetrennt (Fraktion K1), während über die Destillationsbrücke CPMK destilliert wird.

### Bedingungen der Destillation:

- Sumpftemperatur:: 110 - 116 °C
- Kopftemperatur K1:: 79 - 82 °C
- Kopftemperatur K2:: 113 °C
- Rücklauf R1, R/D:: 50 : 1
- Rücklauf R2, R/D:: 1 : 2

- Fraktion K1:: 304,0 g enthaltend 87,2 Gew.- % DHMF,
1,6 Gew.- % CPMK und
11,1 Gew.- % Wasser
- Fraktion K2:: 3 418,8 g enthaltend 99,5 Gew.- % CPMK
- Rückstand:: 245,0 g
Damit beträgt die Gesamtausbeute an 99,5 Gew.-%igem CPMK 93,6 % bezogen auf die Klasse des CPMK im Destillationseinsatz.

## Patentansprüche

1. Verfahren zur Herstellung von hochreinem Cyclopropylmethylketon aus Reaktionsausträgen oder anderen Gemischen, die Cyclopropylmethylketon, 4,5-Dihydro-2-methylfuran, Additionsprodukte des 4,5-Dihydro-2-methylfurans und gegebenenfalls weitere Verunreinigungen enthalten, die aus der Synthese von Cyclopropylmethylketon aus 2-Acetylbutyrolacton resultieren können,
gekennzeichnet durch
zumindest einen kontinuierlich arbeitenden Rektifikationsschritt mit seitlichem Zulauf des durch Rektifikation zu zerlegenden Gemisches in eine Rektifikationsvorrichtung oberhalb des Sumpfes.

2. Verfahren nach Anspruch 1,
gekennzeichnet durch
Sumpftemperaturen bei allen destillativen Behandlungsschritten von kleiner oder gleich 160 °C.

3. Verfahren nach Anspruch 2,
gekennzeichnet durch
Sumpftemperaturen von kleiner oder gleich 140 °C.

4. Verfahren nach Anspruch 3,
gekennzeichnet durch
Sumpftemperaturen von kleiner oder gleich 120 °C.

5. Verfahren nach zumindest einem der vorherigen Ansprüche,
gekennzeichnet durch
ein destillatives Abtrennen von leichter als Cyclopropylmethylketon siedenden Komponenten vor dem Rektifikationsschritt.

6. Verfahren nach zumindest einem der vorherigen Ansprüche 1 bis 4,
gekennzeichnet durch einen einzigen Rektifikationsschritt, bei dem der Zulauf des zu zerlegenden Gemisches in die Rektifiziervorrichtung so angeordnet ist, daß das Verhältnis der Trennstufenanzahl der Verstärkungssäule zur Trennstufenanzahl der Abtriebssäule einen Wert von 1 bis 2 annimmt und bei dem das Cyclopropylmethylketon durch seitliche Entnahme im - bezogen auf die Trennstufenzahl-unteren Drittel der Abtriebssäule oberhalb des Sumpfes gewonnen wird.

7. Verfahren nach zumindest einem der vorherigen Ansprüche,
dadurch gekennzeichnet,
daß die destillative Gewinnung des Cyclopropylmethylketon bei einem Druck von 300 bis 1 050 mbar erfolgt.

8. Verfahren nach zumindest einem der vorherigen Ansprüche,
dadurch gekennzeichnet,
daß die destillative Gewinnung des Cyclopropylmethylketon bei einem Druck von 500 bis 1 030 mbar erfolgt.

9. Verfahren nach zumindest einem der vorherigen Ansprüche,
dadurch gekennzeichnet,
daß die destillative Gewinnung des Cyclopropylmethylketon bei einem Druck von 800 bis 1 020 mbar erfolgt.

## Claims

1. A process for preparing high-purity cyclopropyl methyl ketone from reaction discharges or other mixtures which comprise cyclopropyl methyl ketone, 4,5-dihydro-2-methylfuran, and addition products of 4,5-dihydro-2-methylfuran, with or without other impurities which can result from the synthesis of cyclopropyl methyl ketone from 2-acetylbutyrolactone, characterized by at least one continuous rectification step having a side feed of the mixture to be fractionated by rectification into a rectification apparatus above the bottom.

2. A process according to claim 1, characterized by bottom temperatures in all distillation treatment steps of less than or equal to 160°C.

3. A process according to claim 2, characterized by bottom temperatures of less than or equal to 140°C.

4. A process according to claim 3, characterized by bottom temperatures of less than or equal to 120°C.

5. A process according to at least one of the preceding claims, characterized by components more volatile than cyclopropyl methyl ketone being separated off by distillation prior to the rectification step.

6. A process according to at least one of the preceding claims 1 to 4, characterized by a single rectification step, in which the feed of the mixture to be fractionated into the rectification apparatus is arranged in such a manner that the ratio of the number of theoretical plates of the enrichment column to the number of theoretical plates of the stripping column assumes a value of 1 to 2 and in which the cyclopropyl methyl ketone is obtained by sidestream takeoff in the lower third, based on the number of theoretical plates, of the stripping column above the bottom.

7. A process according to at least one of the preceding claims, characterized in that the cyclopropyl methyl ketone is produced by distillation at a pressure of 300 to 1050 mbar.

8. A process according to at least one of the preceding claims, characterized in that the cyclopropyl methyl ketone is produced by distillation at a pressure of 500 to 1030 mbar.

9. A process according to at least one of the preceding claims, characterized in that the cyclopropyl methyl ketone is produced by distillation at a pressure of 800 to 1020 mbar.

## Revendications

1. Procédé de préparation de la cyclopropylméthylcétone très pure à partir de décharges de réaction ou autres mélanges qui renferment de la cyclopropylméthylcétone, du 4,5-dihydro-2-méthylfurane, des produits d'addition du 4,5-dihydro-2-méthylfurane, et éventuellement d'autres impuretés, qui peuvent résulter de la synthèse de la cyclopropylméthylcétone à partir de la 2-acétylbutyrolactone,
caractérisé par
au moins une étape de rectification travaillant en continu avec une amenée latérale du mélange à fractionner par rectification dans un dispositif de rectification au-dessus du pot.

2. Procédé selon la revendication 1,
caractérisé par
des températures de pot pour toutes les étapes de traitement par distillation, inférieures ou égales à 160°C.

3. Procédé selon la revendication 2,
caractérisé par
des températures de pot inférieures ou égales à 140°C.

4. Procédé selon la revendication 3,
caractérisé par
des températures de pot inférieures ou égales à 120°C.

5. Procédé selon au moins l'une quelconque des revendications précédentes,
caractérisé par
une séparation par distillation des composants bouillant plus facilement que la cyclopropylméthylcétone, avant l'étape de rectification.

6. Procédé selon au moins l'une quelconque des revendications précédentes 1 à 4,
caractérisé par
une étape de rectification unique, dans laquelle l'amenée du mélange à fractionner est disposée dans le dispositif de rectification, de telle sorte que le rapport du nombre d'étages de séparation de la colonne de concentration au nombre d'étages de séparation de la colonne de sortie, prend une valeur de 1 à 2, et que la cyclopropylméthylcétone est obtenue au-dessus du pot par soutirage latéral dans le tiers inférieur rapporté au nombre d'étages de séparation de la colonne de sortie.

7. Procédé selon au moins l'une quelconque des revendications précédentes,
caractérisé en ce que
la production par distillation de la cyclopropylméthylcétone s'effectue à une pression de 300 à 1050 mbars.

8. Procédé selon au moins l'une quelconque des revendications précédentes,
caractérisé en ce que
la production par distillation de la cyclopropylméthylcétone s'effectue à une pression de 500 à 1030 mbars.

9. Procédé selon au moins l'une quelconque des revendications précédentes,
caractérisé en ce que
la production par distillation de la cyclopropylméthylcétone s'effectue à une pression de 800 à 1020 mbars.
